(19) Europäisches Patentamt
European Patent Office
Office européen des brevets

(11) Publication number: 0 050 885
B1

(12) # EUROPEAN PATENT SPECIFICATION

(45) Date of publication of patent specification: 18.07.84

(21) Application number: 81201097.3

(22) Date of filing: 05.10.81

(51) Int. Cl.[3]: C 07 C 93/06, C 07 C 103/00, C 07 C 103/38, C 07 C 121/75, C 07 C 91/16, C 07 D 209/34

(54) Process for preparing 1-amino-3-aryloxy-2-propanols and 1-amino-2-aryl-2-ethanols.

(30) Priority: 16.10.80 IT 2537380

(43) Date of publication of application:
05.05.82 Bulletin 82/18

(45) Publication of the grant of the patent:
18.07.84 Bulletin 84/29

(84) Designated Contracting States:
AT BE CH DE FR GB IT LI LU NL SE

(56) References cited:
JOURNAL OF ORGANIC CHEMISTRY, vol. 43, no. 10, May 12, 1978, T.L. LEMKE et al.: "Synthesis of Methylaryloxypropanolamines", pages 2079-2082

CHEMICAL ABSTRACTS, vol. 76, no. 20, May 15, 1972, page 44, abstract 11407x, COLUMBUS, OHIO (US) R.A. RODINOV et al.: "Curing of epoxy resins by complexes of boron trifluoride with amines"

(73) Proprietor: BLASCHIM S.p.A.
Via Vittor Pisani, 28
I-20124 Milano (IT)

(72) Inventor: Malguzzi, Romualdo
Via Petrarca
I-20078 San Colombano Al Lambro (Milano) (IT)
Inventor: Giordano, Claudio
Via Carlo Porta, 16
I-20052 Monza (Milano) (IT)

(74) Representative: Marchi, Massimo et al,
c/o CON.P.I. Corso Buenos Aires, 64
I-20124 Milano (IT)

EP 0 050 885 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to the preparation of 1-amino-3-aryloxy-2-propanols and 1-amino-2-aryl-2-ethanols by reacting a 1,2-epoxy-3-aryloxy-propane and, respectively, a 1,2-epoxy-2-aryl-ethane with a primary or secondary amine in the presence of a catalytic amount of a Lewis acid.

1-amino-2-aryl-2-ethanols and 1-amino-3-aryloxy-2-propanols are widely used as drugs; examples of such compounds include acetobutolol, alprenolol, atenolol, bevatolol, bufetalol, bufuralol, bunitrolol, carazolol, carteolol, dichloroisoproterenol, isoproterenol, levobunolol, metopropolol, mepindolol, nadolol, oxprenolol, penbutolol, pindolol, practolol, pronethalol, propanol, sotalol, timolol, tiprenolol, tolamolol.

It is known that 1-amino-3-aryloxy-2-propanols and 1-amino-2-aryl-2-ethanols may be prepared by reacting an amine with a 1,2-epoxy-3-aryloxy-propane and, respectively, with a 1,2-epoxy-2-aryl-ethane; good yields are obtained when the reaction is carried out at high temperature, usually at the boiling temperature of the reaction mixture and in a sealed vessel.

When the preparation is carried out at the boiling temperature in an opened vessel it is necessary to combine the reaction vessel with expensive equipments in order to capture the amine which flows out

When the reaction is carried out in a sealed vessel, it is impossible to watch the progress of the reaction and the process implies the well-known risks connected with the high pressure. Now it has been found that the above difficulties may be overcome without affecting the yields when the reaction is carried out at room temperature in the presence of a Lewis acid.

According to a preferred embodiment of this invention the Lewis acids are selected from the group comprising the metal salts and the metal oxydes such as $FeCl_2$, $FeCl_3$, $AlCl_3$, $Al_2O_3$, $BF_3$, $MgI_2$, $MgBr_2$, $MgCl_2$, $CuSO_4$, $CuCl_2$, $Cu_2O_3$, $Cu(OCOCH_3)_2$, $NiCl_2$, $CoCl_2$, $ZnCl_2$, $ZnBr_2$, $SnCl_2$, $SnCl_4$, $SbCl_3$, $MnCl_2$, $Hg_2Cl_2$, $HgCl_2$, $BaCl_2$ $Al(C_3H_7O)_3$ and $CaCl_2$.

The catalyst may be introduced directly into the reaction medium; alternatively it is formed "in situ".

The Lewis acid is preferably used in catalytic amount; larger quantities do not afford appreciable advantages.

When the epoxyde is not soluble in the amine a suitable diluent is added to the reaction mixture, example of such diluent include pyridine, picoline, benzene, toluene, methylene chloride, chloroform, carbon tetrachloride, heptane and octane.

The following specific examples are given to enable those skilled in the art to understand more clearly and practice this invention. It should not be considered as a limitation upon the scope of the invention but merely as being illustrative and representative thereof.

### Example 1

3-[p-(2-methoxyethyl)-phenoxy]-1,2-epoxy-propane (90 g; 0.46 mol) is added to $FeCl_3$ (3.5 g; 0.02 mol) solution in isopropylamine (270 g; 4.57 mol). The solution is stirred at room temperature for 6 hrs. Isopropylamine is removed by evaporation and the residue is dissolved in toluene (300 ml).Toluene solution is extracted with diluted hydrochloric acid (1 N). The aqueous phase is basified with sodium hydroxyde (1 N) and extracted with toluene (300 ml × 3); by distilling off the solvent. Metroprolol is obtained.

Yield: 80 g, corresponding to 70% of the theoretical amount.

Metoprolol (20 g; 0.075 mol) is added to hot acetonitrile (100 ml) under stirring; d-tartaric acid (5.62 g; 0.0375 mol) is added to the hot reaction mixture under stirring The reaction mixture is then kept under reflux and under stirring for 1 hour; after cooling the precipitate is collected by suction and dried at 80°C under reduced pressure.

It is so obtained Metoprolol tartrate; 24.85 g (96.98% of the theoretical amount). m.p. 119°—122°C.

Using different Lewis acid the following results have been obtained:

$AlCl_3$ (3.5 g; 0.03 mol); Yield, 96%
$CuCl_2$ (3.5 g; 0.03 mol); yield, 80%
$MgCl_2$ (3.5 g; 0.04 mol); yield, 82%
$BF_3$ (3.5 g; 0.05 mol); yield, 92%
$MgI_2$ (13.9 g; 0.05 mol); yield 98%
$Al(C_3H_7O)_3$ (10.2 g; 0.05 mol); yield, 78%
$NiBr_2$ (10.2 g; 0.05 mol); yield, 68%
$Co(CH_3COO)_3$ (11.8 g; 0.05 mol); yield, 64%
CuO (4 g; 0.05 mol); yield, 62%

### Example 2

3-(1-naphtoxy)1,2-epoxy-propane (20 g; 0.1 mol) is added to $AlCl_3$ (1.3 g; 0.01 mol) solution is isopropylamine (59 g; 1 mol). The solution is stirred at 15°—20°C for 5 hours.

Isopropylamine is removed by evaporation and the residue is dissolved in methylene chloride (70 ml).

Methylene chloride solution is extracted with diluted hydrochloric acid (1 N). The aqueous phase is basified with sodium hydroxyde (1 N) and extracted with methylene chloride (70 ml × 3); after removal of the solvent Propanolol is obtained. m.p. = 94°—95°C

Yield: 22.3 corresponding to 86% of the theoretical amount.

Example 3

3-[(p-carbamoylmethyl)-phenoxy]-1,2-epoxy-propane (20.7 g; 0.1 mol) is added to a $AlCl_3$ (1.3 g; 0.01 mol) solution in isopropylamine (59 g; 1 mol) and pyridine (280 mol). The solution is stirred at 15°—20°C for 7 hours.

Isopropylamine and pyridine are removed by evaporation; the residue is treated with hydrochloric acid (1 N) and filtered.

The aqueous solution is basified with sodium hydroxyde and filtered; the solid is collected and dried at 100°C under reduced pressure.

Yield: 23.3 g (87.5% of the theoretical amount) of Atenolol melting at 119°—122°C.

With the procedures of Examples from 1 to 3 the following products have been prepared:

Acebutolol
Pindolol
Practolol
Bunitrolol
Pronethalol

## Claims

1. Process for preparing a product having the following formula:

$$Ar—(OCH_2)n—CHOH—CH_2— NR_1R_2$$

where n is 0 or 1

Ar is an aryl radical selected from the group comprising a substituted phenyl ring, a phenyl fused with a heterocyclic ring, a substituted phenyl fused with a heterocyclic ring, a phenyl fused with a substituted heterocyclic ring, a phenyl fused with a cycloaliphatic ring, a phenyl fused with a substituted cycloaliphatic ring, a substituted phenyl fused with a cycloaliphatic ring, a heterocyclic ring, a substituted heterocyclic ring, a naphtyl ring and a substituted naphtyl ring; and

$R_1$ is H or an alkyl radical having from 1 to 5 carbon atoms: and

$R_2$ is an alkyl radical having from 1 to 5 carbon atoms or

$$—CH_2—CH_2(O)_n—AR_1$$

where n has the above mentioned meaning and $Ar_1$ is a substituted phenyl ring which comprises the reaction of a product having the following formula

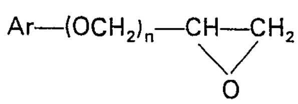

with an amine of formula $HNR_1R_2$ where Ar, n, $R_1$ and $R_2$ have the above mentioned meanings at room temperature and in the presence of a catalytic amount of a Lewis acid.

2. Process according to claim 1 wherein the Lewis acid is a metal salt or a metal oxyde.

3. Process according to claim 1 wherein the amine is a primary amine.

4. Process according to claim 1 and 3 wherein the amine is selected from the group comprising isopropylamine and sec. butylamine.

5. Process according to claim 1 wherein the reaction is carried out in the presence of a diluent selected from the group comprising pyridine, picoline, benzene, toluene, methylene chloride, chloroform, carbon tetrachloride, heptane and octane.

## Revendications

1. Procédé pour la préparation d'un produit répondant à la formule suivante:

$$Ar—(OCH_2)_n—CHOH—CH_2—NR_1R_2$$

dans laquelle:

n est égal à 0 ou 1

Ar est un radical aryle choisi dans le groupe constitué par un noyau phénylique substitué, un phényle condensé avec un noyau hétérocyclique, un phényle substitué condensé avec un noyau hétérocyclique, un phényle condensé avec un noyau hétérocyclique substitué, un phényle condensé avec un noyau cycloaliphatique, un phényle condensé avec un noyau cycloaliphatique substitué, un phényle substitué condensé avec un noyau cycloaliphatique, un noyau hétérocyclique, un noyau hétérocyclique substitué, un noyau naphtylique et un noyau naphtylique substitué;

$R_1$ est H ou un radical alcoyle contenant 1 à 5 atomes de carbone; et

$R_2$ est un radical alcoyle contenant 1 à 5 atomes de carbone ou

$$-CH_2-CH_2(O)_n-Ar_1$$

dans laquelle n a la signification donnée ci-dessus et $Ar_1$ est un noyau phénylique substitué, consistant à faire réagir un produit répondant à la formule suivante:

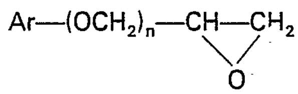

avec une amine de formule $HNR_1R_2$, Ar, n, $R_1$ et $R_2$ ayant les significations données ci-dessus, la température ambiante et en présence d'une quantité catalytique d'un acide de Lewis.

2. Procédé selon la revendication 1, caractérisé en ce que l'acide de Lewis est un sel de métal ou un oxyde de métal.

3. Procédé selon la revendication 1, caractérisé en ce que l'amine est une amine primaire.

4. Procédé selon la revendication 1 ou 3, caractérisé en ce que l'amide est choisie dans le groupe constitué par l'isopropylamine et la secundobutylamine.

5. Procédé selon la revendication 1, caractérisé en ce que la réaction est menée en présence d'un diluant choisi dans le groupe constitué par la pyridine, la picoline, le benzène, le toluène, le chlorure de méthylène, le chloroforme, le tétrachlorure de carbone, l'heptane et l'octane.

## Patentansprüche

1. Verfahren zur Herstellung von Verbindungen der allgemeinen Formel:

$$Ar-(OCH_2)_n-CHOH-CH_2-NR_1R_2$$

worin

n für 0 oder 1 steht,

Ar einen Arylrest bedeutet, ausgewählt aus der Gruppe der folgenden Reste: einem substituierten Phenylring; einem Phenylring, der mit einem hetrocyclischen Ring kondensiert ist; einem substituierten Phenylring, der mit einem heterocyclischen Ring kondensiert ist; einem Phenylring, der mit einem substituierten heterocyclischen Ring kondensiert ist; einem Phenylring, der mit einem cycloaliphatischen Ring kondensiert ist; einem Phenylring, der mit einem substituierten cycloaliphatischen Ring kondensiert ist; einem substituierten Phenylring, der mit einem cycloaliphatischen Ring kondensiert ist; einem heterocyclischen Ring; einem substituierten heterocyclischen Ring; einem Naphtylring und einem substituierten Naphtylring; und

$R_1$ ein Wasserstoffatom oder einen Alkylrest mit 1—5 Kohlenstoffatom bedeutet, und

$R_2$ für einen Alkylrest mit 1 bis 5 Kohlenstoffatomen oder für

$$-CH_2-CH_2(O)_n-Ar_1$$

worin n die oben angegebene Bedeutung besitzt und $Ar_1$ einen substituierten Phenylring bedeutet, steht, dadurch gekennzeichnet, daß man eine Verbindung der allgemeinen Formel

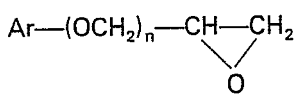

mit einem Amin der Formel $HNR_1R_2$, worin Ar, n, $R_1$ und $R_2$ die oben angegebenen Bedeutungen besitzen, bei Raumtemperatur und in Gegenwart einer katalytischen Menge einer Lewissäure umsetzt.

2. Verfahren nach Anspruch 1, worin die Lewissäure ein Metallsalz oder ein Metalloxid ist.

3. Verfahren nach Anspruch 1, worin das Amin ein primäres Amin ist.

4. Verfahren nach Anspruch 1 und 3, worin das Amin ausgewählt ist unter Isopropylamin und sec.-Butylamin.

5. Verfahren nach Anspruch 1, worin die Umsetzung in Gegenwart eines Verdünnungsmittels durchgeführt wird, das ausgewählt ist unter Pyridin, Picolin, Benzol, Toluol, Methylenchlorid, Chloroform, Tetrachlorkohlenstoff, Heptan und Octan.